## Europäisches Patentamt

## European Patent Office

## Office européen des brevets

(11) Veröffentlichungsnummer: **0 325 201**
**A2**

# EUROPÄISCHE PATENTANMELDUNG

(21) Anmeldenummer: **89100722.1**

(22) Anmeldetag: **17.01.89**

(51) Int. Cl.⁴: **A61N 1/36**

(30) Priorität: **20.01.88 CH 187/88**

(43) Veröffentlichungstag der Anmeldung:
**26.07.89 Patentblatt 89/30**

(84) Benannte Vertragsstaaten:
**AT CH DE LI**

(71) Anmelder: **ETAMA AG**
**Landstrasse 938**
**FL-9496 Balzers(LI)**

(72) Erfinder: **Frick, Kuno**
**Kreuzstrasse 471**
**FL-9496 Balzers(LI)**

(74) Vertreter: **Büchel, Kurt F., Dr.**
**Patentanwalt Dr. Kurt F. Büchel Bergstrasse**
**297**
**FL-9495 Triesen(LI)**

(54) **Stimulationsgerät zur Verbesserung des Sehvermögens des Sehbehinderten.**

(57) Die Erfindung betrifft ein Stimulationsgerät zu Verbesserung des Sehvermögens von Sehbehinderten durch Applizierung von elektrischen Wechselstrcm- oder Mischstromstimulationssignalen, die direkt über Elektroden (9, 19) an das Augenlid (11) eines Patienten angekoppelt werden. Durch die Einwirkung der Stimulationssignale wird das Gewebe der Augen (14) bzw. der Bereich der Augen (Netzhaut, Nerven, Muskeln usw.) zur Heilung angeregt und das Sehvermögen verbessert. Die Stimulationssignale werden mit einer Spannung zwischen 1,3 und 2,3 Volt, sowie mit einem Stromwert zwischen 350 und 450 mA bei Frequenzen zwischen 200 Hz und 2500 Hz abgegeben. Die Behandlungsdauer beträgt ca. eine bis zwei Stunden pro Tag.

FIG 2

EP 0 325 201 A2

## STIMULATIONSGERAET ZUR VERBESSERUNG DES SEHVERMOEGENS DES SEHBEHINDERTEN

Die Erfindung betrifft ein Stimulationsgerät nach dem Oberbegriff des Anspruches 1.

Ein Stimulationsgerät beruht auf dem Prinzip, dass elektrische Stimulationssignale erzeugt werden, die über eine Ankopplungseinrichtung an das Auge, bzw. zum Auge, bzw. zum Gewebe des Sehorgans - im weitesten Sinne - angekoppelt werden. Unter Gewebe des Sehorgans sind beispielsweise auch Nervenbahnen, Augenhintergrund, Netzhaut, Glaskörper, Muskeln usw. zu verstehen. Durch die durch das Gerät erzeugten Signale werden unterschiedlichste Heilprozesse angeregt, wie z.B. Entzündungsminderung, Nervenkräftigung und -anregung, sowie Blutabsorption usw. Ausserdem wird die Sehbahn stimuliert und, wie praktische Erprobungen des Gerätes gezeigt haben - offensichtlich als Summe der Wirkungen - eine Verbesserung des Sehvermögens insgesamt erreicht.

Ströme und Spannungen der beim Sehprozess über den Sehnerv weitergegebenen Signale sind bekannt, wobei die Spannung bei etwa 0,5 Volt und der Strom bei etwa 0,0075 mA liegt. Bei der Auslegung bekannter Geräte wurde dafür gesorgt, dass Signale dieser Grössenordnung an den Sehnerv abgegeben werden. Mit anderen Worten wurde die Schaltungseinrichtung so ausgelegt, dass das an den Sehnerv angekoppelte Signal bei der Uebertragung und/oder Ankopplung der Signale von der Schaltungseinrichtung zu der Sehbahn die erwähnten physiologischen Werte hat. Ein derartiges bekanntes Gerät ist in der DE-OS 2948267 beschrieben. Die mit einem solchen herkömmlichen Gerät erzielten Wirkungen erstrecken sich jedoch stets nur auf Bereiche direkt in den Sehbahnen und im Sehzentrum.

Niedere Spannungs- oder Stromwerte einzusetzen wurde offensichtlich nie in Erwägung gezogen, da man sich dadurch keine Erfolge versprechen konnte. Bei Anwendung des erfindungsgemässen Gerätes wurde nun aber überraschend festgestellt, dass keinerlei Schäden auftreten und dass die Heilung der verschiedensten Irregularitäten wesentlich umfangreicher und rascher als mit bekannten Geräten erfolgt.

Das Stimulationsgerät kann bei allen Patienten angewendet werden, deren Sehvermögen beeinträchtigt ist, deren Augen (zumindest eines) jedoch vorhanden und zumindest teilweise intakt sind. In Ausnahmefällen kann die Behinderung der Patienten auch darin bestehen, dass die natürliche, optische Einrichtung des Auges operativ durch eine künstliche optische Einrichtung ersetzt worden ist, beispielsweise bei der sogenannten strampelli-Operation. Wesentlich ist die Stärkung des noch vorhandenen aktiven Gewebes durch das Stimulationsgerät.

In allen Fällen wird die Ankopplungseinrichtung auf die geschlossenen Augenlider beider Augen oder auf das geschlossene Lid von dem jeweils zu behandelnden Auge aufgesetzt. Die Signale werden dann von den Elektroden über das Augenlid auf den Augapfel, die Augenflüssigkeit, an die Sehbahn, bzw. an sämtliches Gewebe im Bereich das Auges weitergegeben. Die Anordnung der Elektroden ist dabei ein zu berücksichtigender Faktor.

Bei Patienten, die beide Augäpfel besitzen, ist es nicht immer erforderlich, dass die Signale an beiden Augen angekoppelt werden. Es genügt vielmehr, wenn die Signale nur von einem Auge her angekoppelt werden, soferne einzig die Behandlung der Sehbahnen indiziert ist.

Die Stimulationssignale können, wie bei bekannten Geräten, pulsierende Gleichstrom- oder Mischstromsignale sein. Die Signale können in weiten Grenzen variiert werden. Die Stimulationssignale werden aber optimal von einem astabilen Multivibrator als Rechtecksignale erzeugt.

Durch Änderung der Signalfrequenz und durch Änderung der Amplitude können die Stimulationssignale auf die Bedürfnisse des einzelnen Patienten eingestellt werden. In gewissem Umfang kann diese Einstellung vom Patienten selbst vorgenommen werden. Er variiert die Impulsfrequenz und die Amplitude der Signale so lange, bis er ein angenehm prickelndes Gefühl im Augenbereich verspürt. In manchen Fällen kann sich der Patient an einer grauen bis hellgrauen Fläche orientieren, die als scheinbares Bild, bedingt durch die Impulse entsteht. Je stärker die Stimulation ist, desto heller ist der Grauton, den der Patient zu sehen glaubt. In manchen Fällen tritt sogar eine weisse Fläche in Erscheinung.

Die Elektroden der Kopplungseinrichtung sind bevorzugt direkt mit der elektrischen Schaltungseinrichtung verbunden. Die Übertragung von der Schaltungseinrichtung zu der Ankopplungseinrichtung kann aber auch, wie an sich bekannt, über einen Sender oder einen Empfänger erfolgen. Im ersteren Fall geht bei der Übertragung wesentlich weniger Energie verloren und die Schaltung ist wesentlich kleiner.

Bei Patienten, die noch beide Augäpfel haben, ist vorteilhaft, wenn das Stimulationsgerät in ein brillenähnliches Gestell eingebaut ist, wobei die Ausgangselektroden auf, einer Platte angeordnet sind, die durch ein federndes Element gegen die geschlossenen Augenlider gedrückt wird. Bevorzugt ist jedoch die Anordnung an der Rückseite einer Augenabdeckklappe, da dadurch während der Behandlung gleichzeitig das Auge abgedeckt ist, womit eine Überreizung der Netzhaut vermieden wird. In einem solchen Fall ist das Stimulationsgerät vorteilhafterweise aus einem Stück mit der Koppeleinrichtung ausgebildet.

Die Behandlung mit dem Gerät erfolgt so, dass der Patient das Gerät etwa ein bis zwei Stunden pro Tag anwendet, wobei die durch das Gerät verbesserte Sehkraft in der Regel langsam gesteigert wird und dann bei einem noch optimal zu erzielenden Stand konstant bleibt, sodass der Patient in der Zeit zwischen der Stimulationsanwendung besser sehen kann.

Weitere Merkmale und Ausführungsbeispiele des erfindungsgemässen Stimulationssgerätes werden nun und anhand von Skizzen beschrieben. Es zeigen:

Fig. 1 das Schaltbild einer Ausführungsform des erfindungsgemässen Stimulationsgerätes;

Fig. 2 einen schematischen Schnitt durch das Stimulationsgerät und einen Teil des Auges eines Patienten;

Fig. 3 und 4 eine Augenabdeckklappe mit einer Kopplungseinrichtung;

Fig. 5 das Schaltbild eines einstellbaren Taktgenerators und die;

Fig. 6 einen automatischen Taktgenerator.

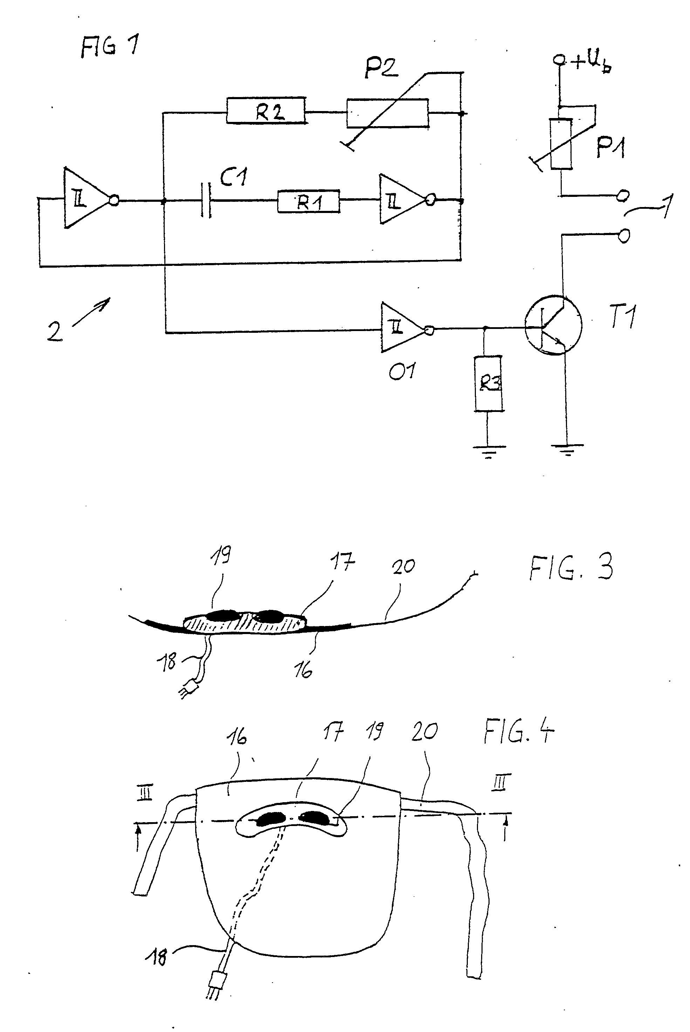

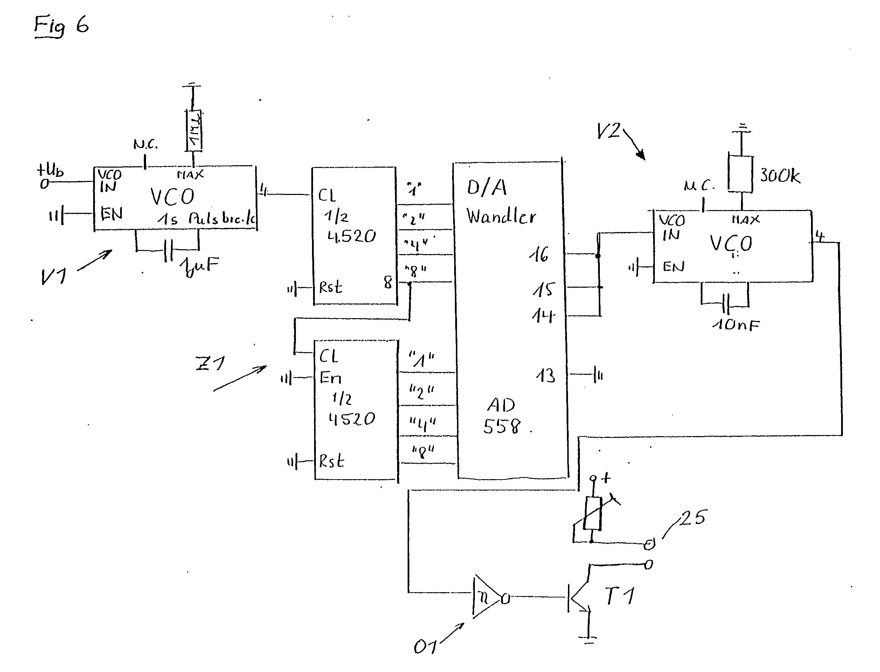

Die Fig. 1 zeigt einen astabilen Multivibrator, dessen Ausgang 1 einer Koppeleinrichtung, bzw. Elektroden 9 wie beispielsweise in Fig. 3 und 4 dargestellt, zugeführt ist. Als Versorgungsspannung dient eine Gleichspannung, beispielsweise von einer nicht näher dargestellten Batterie, die bei U + in die Schaltung einfliesst. Unterhalb des Einganges von U + befindet sich ein Regelpotentiometer P1, mit dessen Hilfe die Amplitudenstärke des Ausgangssignales variiert werden kann. Unterhalb des Regelpotentiometers P1 befindet sich der erwähnte Ausgang 1 und darunter ein Ausgangstransistor T1, der mit seinem Emitter auf Masse liegt und an seiner Basis vom eigentlichen Multivibrator angesteuert wird. Die Schaltung 2 des Multivibrators ist einem Durchschnittsfachmann bekannt, und daher nicht näher beschrieben. Sie verfügt über Widerstände R1, R2 und R3, einen Kondensator C1 sowie zusätzlich in Serie zu dem Widerstand R2 über ein zweites Regelpotentio meter P2, mit dessen Hilfe die Frequenz des Rückkopplungskreises der Schaltung 2 verändert werden kann. Die Spannungszuführung zu diesem bekannten Multivibrator ist ebenfalls bekannt und daher nicht dargestellt.

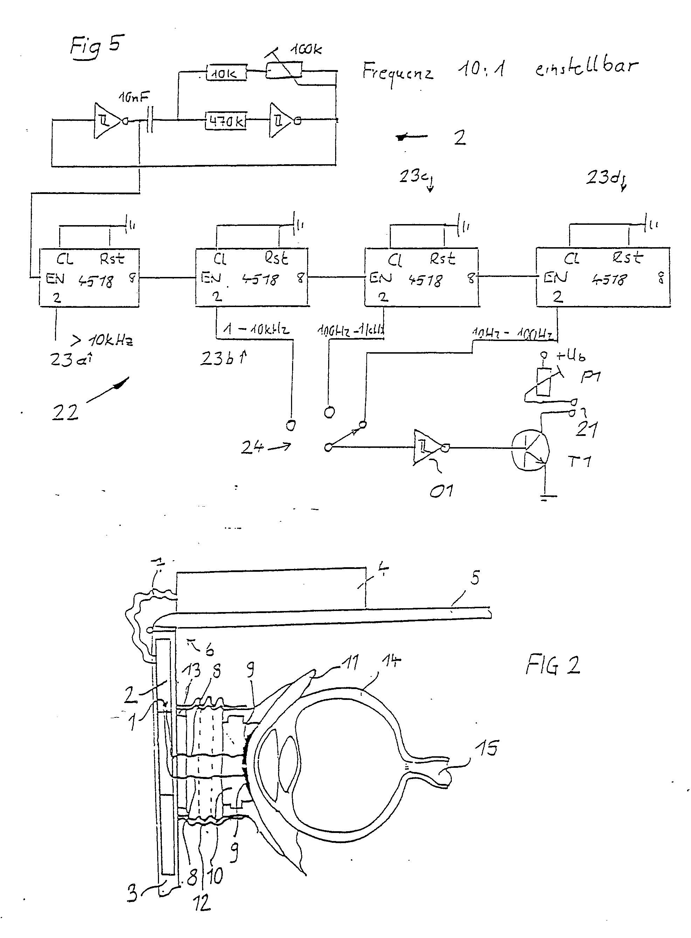

Die Schaltung 2 ist (siehe Fig. 2) in einem brillenartigen Gestell 3 angeordnet. Sie wird dort von einer Batterie 4 versorgt, die in einem Bügel 5 über ein Scharnier 6 mit dem Brillengestell 3 verbunden ist. Die Batterie 4 könnte auch an einem anderen Ort, beispielsweise an einer Umhängetasche untergebracht sein. Sie ist ebenfalls über Stromleitungen 7 mit der Schaltung 2 verbunden. Der Ausgang 1 der Schaltung 2 wird über je einen Draht 8 mit Elektroden 9 verbunden, die in einer Kopplungseinrichtung 10 angebracht sind. Die Kopplungseinrichtung 10 ist ein gegossener Kunststoffkörper, dessen äussere Form der Umgebung eines Augenlides 11 angepasst ist. Sie ist an einem federnden Faltenbalg 12 befestigt, der an seinem anderen Ende mittels eines Flansches 13 an dem Brillengestell 3 befestigt ist. Sobald also das Brillengestell 3 auf den kopf eines Patienten gesetzt ist, berühren die Elektroden 9 federbelastet das Augenlid 11 und ermöglichen so die Uebertragung der Signale beispielsweise an das Auge 14 oder die Nervenbahnen 15.

Das Regulieren der Amplitude und Frequenz des Ausgangsignales erfolgt über nicht mehr dargestellte Drehknöpfe am Brillengestell 3.

In der Fig. 3 und 4 ist im Schnitt bzw. im Aufriss eine Augenabdeckklappe 16 ersichtlich, die eine nierenförmige Kopplungseinrichtung 17 trägt. Sie ist ebenso mit Elektroden 19 ausgestattet und über eine elektrische Anschlussleitung 18 mit dem Stimulationsgerät verbindbar. Ein gummielastisches Halteband 20 ermöglicht das Befestigen der Augenabdeckklappe am Kopf eines Patienten. Die Elektroden 19 sind höchstens 3,5 mm voneinander entfernt.

Das Stimulationsgerät selbst könnte ohne weiteres auch wie in Fig. 2 direkt (in einem Stück) mit der nierenförmigen Kopplungseinrichtung 17 verbunden sein. In einem solchen Fall dient die Leitung 18 zur Stromzuführung.

Aus Fig. 5 ist eine Frequenzteilerschaltung 22 vorgesehen, die aus 4 integrierten Frequenzteilern 23a - d (integrierter Bauteil 4518) und einem Umschalter 24 besteht. Die Frequenzteilerschaltung 22 ist zwischen dem astabilen Multivibrator 2 (vgl. Fig. 1 ) und einem Operationsverstärker 01 vor dem Ausgangstransistor T1 eingebaut. Die Frequenzteiler teilen in die folgenden Frequenzbereiche:
grösser als 10 kHz; 1-10 kHz; 100 Hz-1kHz und 10Hz-100Hz. Der Bereich grösser als 10kHz (des Frequenzteilers 23a) ist nicht mit dem Umschalter 24 verbunden, da dieser Frequenzbereich für die Behandlung uninteressant ist.

Die in der Fig. 6 dargestellte Schaltung erlaubt selbsttätige Frequenzvariationen nach einem voreingegebenen Programm. Dazu dient ein spannungsabhängiger Frequenzgenerator V1, der an seinem Ausgang mit einem integrierten Zähler Z1 verbunden ist. Der Zähler Z1 ist durch einen integrierten Bauteil mit der Nr. 4520 gebildet. Er ist an einem Digital-Analogwandler D1 (AD 558) angeschlossen, dessen Ausgänge an einen weiteren spannungsabhängigen Frequenzgenerator V2 gelegt sind. Der Ausgang des zweiten spannungsabhängigen Frequenzgenerators V2 ist mit dem Operationsverstärker 01 vor dem Transistor T1 verbunden, der wie in den Schaltungen der Fig. 1 und 5 einen Ausgang 25 ansteuert. Durch diese Schaltungsanordnung variiert sich die Frequenz der abgegebenen Impulse ständig selbsttätig, wodurch einen grosse Streuung dieser Impulse bei der Behandlung und damit eine hohe Erfolgsquote gewährleistet ist.

Die Erfindung ist durch die dargestellten Figuren nicht eingeschränkt. Entscheidend sind die Merkmale des Kennzeichens des Anspruches 1 für die erfolgreiche Therapie von sehschwachen Personen.

Am idealsten haben sich Frequenzen zwischen 50 Hz und 2,5kHz erwiesen.

## BEZUGSZEICHENLISTE

1 Ausgang
2 Schaltung des Multivibrators
3 Brillengestell
4 Batterie
5 Bügel
6 Scharnier
7 Stromleitungen
8 Draht
9 Elektroden
10 Kopplunseinrichtung
11 Augenlid
12 Faltenbalg
13 Flansch
14 Auge
15 Nerven
16 Augenabdeckklappe
17 Nierenförmige Kopplunseinrichtung
18 Elektrische Anschlussleitung
19 Elektroden
20 Elastisches Band
21 Ausgang
22 Schaltung
23 Frequenzteiler a - d
24 Umschalter
P1 Regelpotenziometer
U + Versorgungsspannung
T1 Ausgangstransistor
P2 Regelpotenziometer
U1 Operationsverstärker
V1 Spannungsgesteuerter Frequenzgenerator
Z1 Zähler
D1 Digital-Analogwandler
V2 Zweiter spannungsabhänigiger Frequenzgenerator
25 Ausgang

## Ansprüche

1. Stimulationsgerät zur Verbesserung des Sehvermögens eines Sehbehinderten, mit einer Schaltung (2, 22) zur Erzeugung von elektrischen Signalen und mit mindestens 2 Ausgangselektroden (9, 19) für das Übertragen dieser Signale an den Sehbehinderten, dadurch gekennzeichnet, dass die Signale Rechteckimpulse mit einem Spannungswert zwischen 1,3 und 2,3 volt und/oder mit einem Stromwert zwischen 0,002 und 0,003 mA sind, die mit einer variierbaren Frequenz zwischen 50 Hz und 2500 Hz abgebbar sind.

2. Stimulationsgerät nach Anspruch 1, dadurch gekennzeichnet, dass die Schaltung (2, 22) einen astabilen Multivibrator mit einer Ausgangsstufe (Ausgangstransistor T1) und ein Regelpotentiometer (P1) aufweist, durch das die Spannung/Strom-Amplitude veränderbar ist.

3. Stimulationsgerät nach Anspruch 1 oder 2, dadurch gekennzeichnet, dass die Schaltung (2,22) mit den Elektroden (9,19) zusammengebaut ist, und dass die Elektroden (9,19) an einer Kopplungseinrichtung (10,17) ausgebildet sind, die der Umgebung eines menschlichen Augenlides (11) angepasst ist, wobei die Elektroden (9,19) VZ konvex bombiert ausgebildet sind.

4. Stimulationsgerät nach Anspruch 3, dadurch gekennzeichnet, dass die Schaltung (2, 22) und die Elektroden (9, 19) in einen Kunststoffkörper eingegossen sind, der eine Befestigungvorrichtung für das Zusammenwirken mit einer Halterung aufweist.

5. Stimulationsgerät nach Anspruch 4, dadurch gekennzeichnet, dass die Halterung ein elastisches Band (20) ist, über welches mittels Kabel (18) eine Stromzuführung von einer Batterie an die Schaltung vorgesehen ist.

6. Stimulationsgerät nach Anspruch 4 oder 5, dadurch gekennzeichnet, dass die Schaltung (2,22) und die Elektroden (9,19) an der Rückseite einer Augenabdeckklappe (16) vorgesehen sind.

7. Stimulationsgerät nach Anspruch 4 oder 5, dadurch gekennzeichnet, dass die Schaltung (2) und die Elektroden (9) an einem brillenartigen Gestell (3) angeordnet sind, wobei die Elektroden (9) vorzugsweise federbelastet sind.

8. Stimulationsgerät nach einem der vorhergehenden Ansprüche, dadurch gekennzeichnet, dass die Elektroden aus Gold, Platin, oder aus einer gold- oder platinhaltigen Legierung bestehen.

9. Stimulationsgerät nach einem der vorhergehenden Ansprüche, dadurch gekennzeichnet, dass die Elektroden (9) in einem Abstand von 0,5 bis 3,5 mm angeordnet sind.

10. Stimulationsgerät zur Verbesserung des Sehvermögens eines Sehbehinderten nach einem der vorhergehenden Ansprüche, dadurch gekennzeichnet, dass eine weitere Schaltung vorgesehen ist, mittels derer die Amplitude und/oder die Frequenz der Impulse nach einem voreingestellten Programm oder mittels eines Zufallsgenerators laufend selbsttätig variierbar sind.

FIG 1

P2

R2

+U_b

P1

C1

R1

2

O1

R3

T1

1

FIG. 3

19    17    20

18    16

FIG. 4

16    17  19  20

III                              III

18

Fig 5

Frequenz 10:1 einstellbar

FIG 2

Fig 6